# EUROPÄISCHE PATENTANMELDUNG

(11) **EP 3 287 081 A2**
(43) Veröffentlichungstag der Anmeldung: **28.02.2018**
(21) Anmeldenummer: 17191846.9
(22) Anmeldetag: 31.07.2013
(51) Int. Cl.: A61B 10/00, G01N 1/02, A61F 13/38, A61B 10/02, B01L 3/00

(54) **PROBENNAHMEEINRICHTUNG FÜR INSBESONDERE DNA-HALTIGE PROBEN**

(30) Priorität: 07.08.2012 DE 102012015706
(62) Teilanmeldung aus: 13744971.6
(71) Anmelder: Prionics AG, 8952 Schlieren (CH)
(72) Erfinder: Lardi, Eligio, 8400 Winterthur (CH); Hostettler, Bernhard, 8044 Gockhausen (CH); Stamm, Cristoph, 8260 Stein am Rhein (CH); Blaser, Mathias, 8050 Zürich (CH); Rüegg, Andreas, 8053 Zürich (CH)
(74) Vertreter: Meissner Bolte Partnerschaft mbB

(57) **Zusammenfassung**

Probennahmeeinrichtung für insbesondere DNA-haltige Proben mit einem Probensammelbereich, der an dem freien Ende einer länglichen Halteeinrichtung vorgesehen ist, wobei der Probensammelbereich von der Probennahmeeinrichtung abtrennbar ausgebildet ist, und die Halteeinrichtung in Längsrichtung gesehen einen proximalen Abschnitt mit einer sich von seinem distalen Ende mindestens über einen Längenbereich in proximale Richtung erstreckenden inneren Ausnehmung und eine distal fluchtend zu der Ausnehmung ausgerichtete Hülse aufweist, wobei der proximale Abschnitt und die distale Hülse über einen mit geringem Spiel in die innere Ausnehmung und die Hülse eingesetzten Zapfen miteinander verbunden sind, wobei die Maße des Zapfens, der Hülse und der inneren Ausnehmung so aufeinander abgestimmt sind, dass im Normalbetrieb eine stabile Verbindung des proximalen Abschnitts mit der Hülse hergestellt ist und dass der Probensammelbereich an einem länglichen Ansatz angeordnet ist, dessen freies Ende wiederum mit geringem Spiel in das distale Ende der Hülse eingesetzt ist, wobei die Längen der inneren Ausnehmung, der Hülse, des Zapfens und des Ansatzes so aufeinander abgestimmt sind, dass der Zapfen bei proximal gerichteter Verschiebung des Ansatzes relativ zur Hülse vollständig in die Ausnehmung einschiebbar ist.

## Beschreibung

Die Erfindung bezieht sich auf eine Einrichtung nach dem Oberbegriff des Anspruches 1.

Gattungsgemässe Einrichtungen kommen zum Einsatz insbesondere auf dem Gebiet der Forensik aber auch bei anderen z.B. medizinischen Anwendungen. Sie dienen zur Entnahme bzw. Sicherung von Probenmaterial, das dann in einem nachgeschalteten Schritt im Labor analysiert wird.

Derartige zur Probennahme geeignete Einrichtungen werden im Fachgebrauch auch als Swab, Tupfer oder auch Forensik-Stäbchen bezeichnet. Sie weisen einen länglichen, in der Regel stäbchenförmigen Haltebereich auf, mit dem ein am distalen Ende des Haltebereichs angeordneter Probensammelbereich bei der Probennahme bewegt werden kann. Der Probensammelbereich kann z. B. aus einem Wattebausch gebildet sein. Denkbar sind aber auch andere Materialien, z.B. beflockte Vliese etc. Wichtig ist lediglich, dass der Probensammelbereich aus einem Material besteht, das in der Lage ist, die Probe bis zur Bearbeitung im Labor dauerhaft aufzunehmen und dann im Zuge der Analyse bei Behandlung mit geeigneten Puffern etc. wieder freizugeben.

Ein wesentlicher Aspekt der Bearbeitung von z.B. forensischem Probenmaterial ist, wie oben bereits, erwähnt die Analyse im Labor. Es können hier z.B. genetische, immunologische oder sonstige Analysen erfolgen.

In aller Regel wird zur Untersuchung im Labor der Probensammelbereich in ein Gefäß überführt, in dem entweder lediglich die Extraktion der enthaltenen Probe erfolgt, oder aber zusätzlich auch bereits die weitere Aufarbeitung vorgenommen wird. Es kann sich dabei z. B. um so genannte Spin-Baskets, aber auch alle anderen für diesen Zweck vorgesehene Laborgefäße wie Eppendorf-Hütchen etc. handeln.

Im Rahmen der nachgeschalteten Aufarbeitung im Labor ist die längliche Halteeinrichtung störend. Gängigerweise erfolgt daher vor der Bearbeitung eine Abtrennung des Probensammelbereichs von der Halteeinrichtung. Es muss dabei sorgfältig darauf geachtet werden, dass im Rahmen der Abtrennung keine Kontamination des Probensammelbereichs erfolgt. Außerdem ist im Rahmen der Laborlogistik und auch im Hinblick auf die in aller Regel relativ große Anzahl von zu bearbeitenden Proben eine weitgehende Automatisierung gewünscht.

In diesem Zusammenhang sind Probennahmeeinrichtungen bekannt, die einen mit der Halteeinrichtung lösbar verbundene Probensammelbereich aufweisen. Bei einer entsprechenden, aus der EP 1409636 bekannten Einrichtung ist weiterhin vorgesehen, dass das zur Aufarbeitung im Labor eingesetzte Gefäß im Inneren Einrichtungen aufweist, die ein Einführen der Probensammelzone in das Gefäß erlauben, jedoch beim Herausziehen der Halteeinrichtung aus dem Gefäß heraus, den Probensammelbereich festhalten. Bei weiterer Bewegung der Halteeinrichtung wird die Verbindung zwischen ihr und dem Probensammelbereich gelöst und die störende Halteeinrichtung kann separat entfernt werden.

Die bekannte Einrichtung hat allerdings den Nachteil, dass zur automatisierten Abtrennung der Probensammelzone von der Halteeinrichtung nicht nur die Probennahmeeinrichtung entsprechend gefertigt werden muss. Vielmehr müssen auch in dem zur Aufarbeitung vorgesehenen Laborgefäß die oben erwähnten Einrichtungen, die die Probensammelzone festhalten, vorgesehen werden.

Aufgabe der Erfindung ist es daher, eine Probennahmeeinrichtung zu schaffen, die eine automatisierte Trennung von Probensammelzone und Halteeinrichtung erlaubt, ohne dass hierfür spezielle Laborbehälter erforderlich sind.

Gelöst wird die Aufgabe mit einer Probennahmeeinrichtung, die die Merkmale des Anspruchs 1 oder des Anspruchs 3 aufweist.

Erfindungsgemäss weist eine erste Variante der Probennahmeeinrichtung eine längliche Halteeinrichtung auf, die in Längsrichtung gesehen, in einen proximalen Abschnitt und eine distale Hülse unterteilt ist. Der proximale Abschnitt weist dabei eine sich über einen von seinem distalen Ende in proximaler Richtung erstreckende innere Ausnehmung auf, bzw. ist bevorzugt auch als durchgehende Hülse ausgebildet.

Die distale Hülse ist zu der Ausnehmung fluchtend ausgerichtet. Proximaler Abschnitt und Hülse sind über einen mit geringem Spiel sowohl in die innere Ausnehmung als auch in die Hülse einsetzbaren Zapfen miteinander verbunden. Die Abmessungen des Zapfens sowie der inneren Ausnehmung und der Hülse sind dabei so aufeinander abgestimmt, dass bei normalem Einsatz der Einrichtung eine sichere Verbindung der beiden Abschnitte gewährleistet ist. Andererseits muss bei Aufwendung einer definierten höheren, im Probennahmebetrieb unüblichen, Kraft eine Verschiebung des Zapfens in den genannten von ihm verbundenen Teilen möglich sein.

Der Probensammelbereich seinerseits ist an dem einen Ende eines länglichen Ansatzes angeordnet, dessen anderes Ende in das distale Ende der Hülse eingesetzt ist.

Die erfindungsgemäße Probennahmeeinrichtung weist damit eine zweigeteilte Halteeinrichtung auf, deren beide Abschnitte über den Zapfen miteinander verbunden sind. Der Probensammelbereich ist am distalen Ende der Hülse über einen dort in die Hülse eingesetzten Ansatz an der Halteeinrichtung befestigt.

Die Abmessungen der inneren Ausnehmung, der Hülse, des Zapfens sowie des Ansatzes, der den Probensammelbereich hält, sind in einer Ausgestaltung so aufeinander abgestimmt, dass im normalen Betrieb der Probennahmeeinrichtung z.B. alle oder zumindest einige Teile aneinander im Presssitz gesichert sind. Eine weitere Ausgestaltung betrifft eine Sicherung mittels Formschlusseingriff. In dieser Ausgestaltung ist vorgesehen, dass Zapfen und/oder Ansatz in der Hülse und/oder inneren Ausnehmung z.B. mittels einer Rastverbindung gesichert sind. Der Zapfen und/oder Ansatz weisen dazu nach aussen weisende Rastnasen auf, die z.B. unter Federkraft in innere Ausnehmungen bzw. Durchbrüche in der Hülse bzw. inneren Ausnehmung eingreifen. Die Rastnasen sind so gestaltet, dass der Rasteingriff bei proximaler Verschiebung des Ansatzes gelöst wird und eine weitere proximale Verschiebung von Ansatz und Zapfen möglich ist.

Denkbar ist natürlich auch, Kombinationen von Pressicherung und Formschlusssicherung in einer Probennahmeeinrichung vorzusehen.

Erfindungswesentlich ist, dass bei Aufbringung einer einen definierten Wert übersteigenden Kraft, die z.B. in Längsrichtung nach unten auf das proximale Ende der Probennahmeeinrichtung bei fixiertem distalem Ende wirkt, eine Verschiebung der genannten Komponenten möglich ist. In einem solchen Fall wird der proximale Abschnitt zusammen mit der daran angrenzenden distalen Hülse relativ zu dem Ansatz und dem Zapfen verschoben. Der Zapfen bewegt sich dabei von dem Ansatz geschoben in proximaler Richtung in die Ausnehmung des proximalen Abschnittes hinein und gibt, wenn er vollständig in diese Ausnehmung geschoben wurde, die Verbindung zwischen proximalem Abschnitt und Hülse frei. Der Probensammelbereich ist dann zusammen mit der Hülse von dem proximalen Abschnitt der Probennahmeeinrichtung abgetrennt.

Es versteht sich, dass die innere Ausnehmung des oberen Abschnittes so lang ausgebildet werden muss, dass ein vollständiges Hineinschieben des Zapfens möglich ist.

Mit der erfindungsgemäßen Einrichtung ist eine besonders einfache automatisierte Abtrennung der Probensammelzone möglich. Es genügt, die Einrichtung am proximalen Ende zu fixieren und mit dem distalen, den Probensammelzbereich one bildenden Ende in einen Behälter einzuführen, bis dieses am Boden des Gefässes anliegt. Wird dann das proximale Ende weiter in Richtung auf den Boden des Behälters bewegt, dann kommt es zu der oben beschriebenen Relativverschiebung von Ansatz und Zapfen zu den umgebenden Teilen der Halteeinrichtung, mit dem Erfolg, dass die Probensammelzone von dem oberen Abschnitt der Halteeinrichtung abgetrennt wird.

Es versteht sich, dass die Dimensionierung der Abschnitte beliebig gewählt werden kann. Im Hinblick auf die gewünschte Abtrennung im Wesentlichen nur des Probensammelbereichs wird aber wohl in den meisten Anwendungen der proximale Abschnitt deutlich länger ausgebildet als die sich distal daran anschließende Hülse.

Um ein unbeabsichtigtes Abtrennen des Probensammelbereichs bei der Probennahme, z. B. durch zu starkes Aufdrücken, zu verhindern, kann in einer vorteilhaften Ausgestaltung eine in den oberen Abschnitt einsetzbare, die innere Ausnehmung sperrende Begrenzung vorgesehen werden. Diese Begrenzung kann im Labor entfernt werden, woraufhin sich dann die oben beschriebene Abtrennung der Probensammelzone vornehmen lässt.

Wird eine Halteeinrichtung verwendet, bei der auch der obere Abschnitt als durchgehende Hülse ausgebildet ist, dann ist es auch denkbar, den zur Verbindung der beiden Abschnitte vorgesehenen Zapfen so lang auszugestalten, dass er bis an das proximale Ende des proximalen Abschnittes reicht und dort z. B. eine Kappe oder dergleichen vorzusehen, die ein Verschieben des Zapfens in proximaler Richtung behindert. Erst nach Entfernen der Kappe kann dann wiederum die erwähnte Abtrennung der Sammelzone erfolgen.

Bei weiteren Varianten einer erfindungsgemäßen Probennahmeeinrichtung kann vorgesehen sein, dass die Halteeinrichtung eine innere stabförmige Einrichtung aufweist, an deren distalem Ende der Probensammelbereich lösbar angeordnet ist. Weiterhin weist die Halteeinrichtung eine äußere, die innere stabförmige Einrichtung mindestens teilweise in Längs- und Umfangsrichtung umgebende Einrichtung auf. Innere und äußere Einrichtung sind relativ zueinander verstellbar, wobei vorgesehen ist, dass durch eine definierte Relativbewegung von beiden Einrichtungen der Probensammelbereich von der Halteeinrichtung abgetrennt wird.

In einer Ausgestaltung ist die innere Einrichtung ein Stäbchen mit zwei Abschnitten, die über eine Sollbruchstelle miteinander verbunden sind. Die äußere Einrichtung ist ein das Stäbchen in Längsrichtung teilweise umgebendes Röhrchen. Weiterhin ist vorgesehen, dass Stäbchen und Röhrchen distal zu der Sollbruchstelle in verdrehsicherem Eingriff stehen. Wird nun am proximalen Ende des Röhrchens gedreht, so wird aufgrund der distalen Verdrehsicherung zwischen Röhrchen und Stäbchen die Sollbruchstelle zerstört und der sich distal davon erstreckende Teil des Stäbchens, der den Probensammelbereich trägt, wird abgelöst.

Bei einer weiteren Ausgestaltung ist wiederum ein inneres Stäbchen und ein das Stäbchen teilweise umgebendes Röhrchen vorgesehen. Das Stäbchen besteht aus zwei Abschnitten, einem distalen, den Probensammelbereich tragenden und einem daran angrenzend angeordneten proximalen Abschnitt, der nach oben übersteht. In Betrieb ist die Trennstelle zwischen distalem und proximalem Abschnitt des Stäbchens von dem äußeren Röhrchen umgeben. Um zu verhindern, dass der distale Abschnitt herausfällt, kann zwischen diesem und dem Röhrchen ein Formschlusseingriff vorgesehen sein.

Zur Trennung wird der proximale, nach oben überstehende Bereich des Stäbchens relativ gesehen in das umgebende Röhrchen hineingedrückt. Denkbar ist dass das Stäbchen bewegt und das Röhrchen fixiert wird. Genauso gut ist es aber auch möglich das Röhrchen gegenüber einem fixierten Stäbchen nach oben zu verschieben. In beiden Fällen wird die Trennstelle zwischen distalem und proximalem Abschnitt nach unten aus dem Röhrchen herausbewegt, wodurch der Probensammelbereich zusammen mit dem distalen Bereich des Stäbchens von der Halteeinrichtung abgetrennt wird.

Es versteht sich, dass der oben erwähnte Formschluss zwischen distalem Bereich des Stäbchens und der Hülse so gewählt sein muss, dass er ohne übermäßigen Kraftaufwand beim Eindrücken des Stäbchens aufgehoben werden kann.

Hier sind unterschiedliche, in den nachfolgenden Figuren (4, 5ab) beschriebene Möglichkeiten denkbar, wie der distale Abschnitt des Stäbchens in dem Röhrchen aufgenommen ist.

Eine weitere Variante schließlich sieht vor, dass die Halteeinrichtung wiederum ein insbesondere inneres Stäbchen aufweist, und eine äußere, parallel dazu ausgerichtete und das Stäbchen zumindest teilweise umgebende Einrichtung. Äußere Einrichtung und Stäbchen sind an ihrem distalen Ende im Presssitz in eine Aufnahme eingesetzt, die an ihrem anderen Ende den Probensammelbereich trägt. Wird nun durch Relativbewegung zwischen innerer und äußerer Einrichtung die äußere Einrichtung aus dem Ansatz herausgezogen, so ist die Fixierung wiederum aufgelöst und der Ansatz sowie der Probensammelbereich werden automatisch abgetrennt.

Im Folgenden soll die Erfindung anhand mehrerer Abbildungen näher erläutert werden.

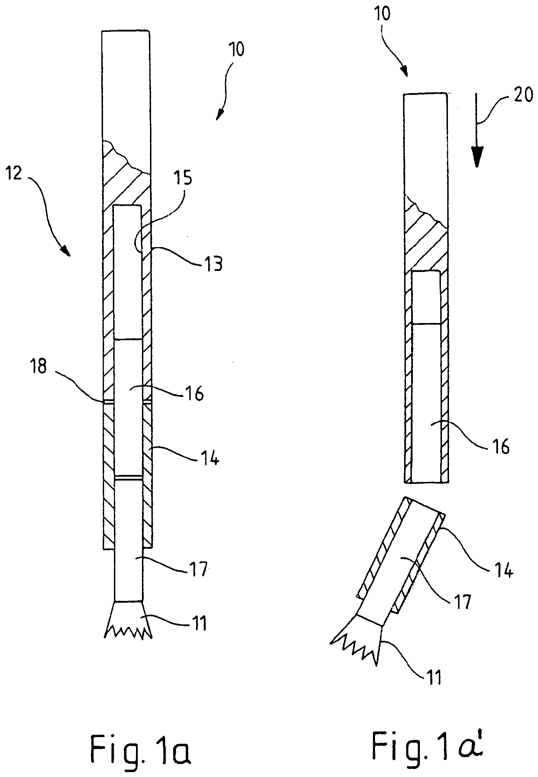

- Fig. 1a: zeigt ein Ausführungsbeispiel der erfindungsgemäßen Vorrichtung gemäss der ersten Variante in zusammengesetztem Zustand,
- Fig. 1a': zeigt dieselbe Einrichtung in getrenntem Zustand.
- Fig. 1b: zeigt ein weiteres Ausführungsbeispiel der erfindungsgemäßen Vorrichtung gemäss der ersten Variante in zusammengesetztem Zustand.
- Fig. 2: zeigt eine Probennahmeeinrichtung gemäss einer weiteren Variante, bei der die Abtrennung des Probensammelbereiches durch Verdrehung einer inneren Einrichtung gegenüber einer äußeren Einrichtung der Halteeinrichtung erfolgt,
- Fig. 3: zeigt eine Probennahmeeinrichtung gemäss einer weiteren Variante, bei der die Abtrennung des Probensammelbereiches durch Vorschieben einer inneren Einrichtung gegenüber einer äußeren Einrichtung der Halteeinrichtung erfolgt,
- Fig. 4: zeigt eine alternative Ausgestaltung des proximalen Endes der in Fig. 3 gezeigten Probennahmeeinrichtung,
- Fig. 5a/5b: zeigen eine alternative Ausgestaltung des distalen Endes der in Fig. 3 gezeigten Probennahmeeinrichtung, und
- Fig. 6: zeigt eine weitere Ausgestaltung einer Probennahmeeinrichtung gemäss einer weiteren Variante.

Fig. 1a zeigt eine Probennahmeeinrichtung 10 mit einem Probensammelbereich 11 und einer Halteeinrichtung 12. Die Halteeinrichtung 12 weist einen oberen Abschnitt 13 auf und eine distal dazu ausgerichtete und angeordnete Hülse 14. In dem proximalen Abschnitt ist eine innere Ausnehmung 15 vorgesehen, die im Wesentlichen dieselben Abmessungen aufweist, wie die inneren Abmessungen der Hülse 14. Die Verbindung der beiden Abschnitte 13 und 14 erfolgt mittels eines mit geringem Spiel sowohl in die innere Ausnehmung 15 als auch in die Hülse 14 eingesetzten Zapfen 16. Der Probensammelbereich 11 seinerseits ist über einen Ansatz 17 mit in der Regel entsprechenden Abmessungen wie der Zapfen 16 in den distalen Endbereich der Hülse 14 eingesetzt. Die Abmessungen des Ansatzes 17, des Zapfens 16, sowie der inneren Ausnehmung 15 und der Hülse 14 sind so gewählt, dass im Normalbetrieb eine stabile Verbindung aller Komponenten miteinander hergestellt ist. Um sicherzugehen, dass bei unsachgemäßer Betätigung keine ungewollte Trennung der Probennahmeeinrichtung 10 im Bereich 18 zwischen proximalem Abschnitt 13 und Hülse 14 eintritt, kann otptional eine nicht dargestellte Sperreinrichtung vorgesehen sein, die ein Verschieben des Zapfens 16 in proximaler Richtung begrenzt. Auf diese Weise wird verhindert, dass der Zapfen unbeabsichtigt über den Bereich 18 in die Ausnehmung 15 verschoben werden kann.

Die Sperreinrichtung ist nicht zwingend erforderlich. Es ist im Rahmen der üblichen Kunststofffertigungsprozesse kein Problem, die genannten Komponenten der Probennahmeeinrichtung mit so genau aufeinander abgestimmten Abmessungen herzustellen, dass auch im Normalbetrieb eine sichere Verbindung aller Komponenten durch z. B. den Zapfen gewährleistet ist und das Risiko, dass dieser unbeabsichtigt in die Ausnehmung verschoben wird, nahezu ausgeschlossen ist. Denkbar ist auch die angesprochenen Komponenten über bezüglich ihrer Halteeigenschaften gut einstellbare Formschlussverbindungen im Normalbetrieb aneinander zu sichern.

Fig. 1a' zeigt nun den Zustand der Probennahmeeinrichtung 10 mit abgetrennter Probensammelvorrichtung 11. Zur Abtrennung wird z. B. das proximale Ende des proximalen Abschnittes 13 mit einer in Pfeilrichtung 20 wirkenden Kraft beaufschlagt, während das distale Ende der Einrichtung, d. h. der Probensammelbereich 11, ortsfest auf z.B. dem Boden eines nicht dargestellten Laborgefäßes aufsitzt. Bei Bewegung des proximalen Abschnittes 13 in Richtung des Pfeiles 20 wird der der Ansatz 17 in proximaler Richtung in die Hülse 14 hineinbewegt und verschiebt den Zapfen 16 nach Kontaktierung vollständig in die innere Ausnehmung 15 des proximalen Abschnittes 13. In dem Moment, wo der Zapfen 16 nicht mehr den Grenzbereich 18 zwischen oberem Abschnitt 13 und Hülse 14 abdeckt, ist die Verbindung in diesem Bereich aufgehoben und die Hülse 14 zusammen mit dem eingesetzten Ansatz 17 und des Probensammelbereichs 11 trennen sich automatisch von dem Rest der Probennahmeeinrichtung 10.

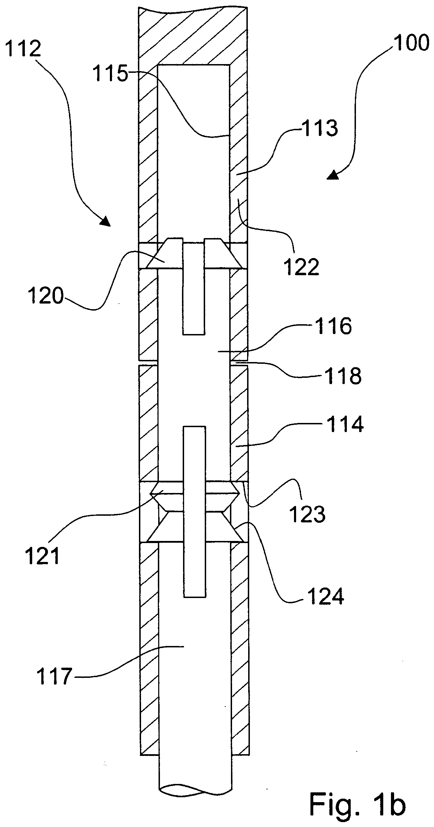

Fig. 1b zeigt eine Probenahmeeinrichtung 100 mit einer nicht dargestellten von einem Ansatz 117 gehaltenen Probensammelvorrichtung und einer Halteeinrichtung 112. Analog zu Fig. 1a weist auch die Halteeinrichtung 112 einen oberen Abschnitt 113 und eine dazu distal ausgerichtete und fluchtend angeordnete Hülse 114 auf.

In dem proximalen Abschnitt ist wiederum eine innere Ausnehmung 115 vorgesehen, die im wesentlichen dieselben Abmessungen aufweist, wie die inneren Abmessungen der Hülse 114. Die Verbindung der beiden Abschnitte 113 und 114 erfolgt mit einem in beide Abschnitte einsetzbaren Zapfen 116.

Abweichend zu Fig. 1a weist der Zapfen 116 an seinen beiden Enden Rastnasen 120 und 121 auf, die jeweils mit in der inneren Ausnehmung 113 und der Hülse 114 vorgesehenen Durchbrüchen 122 und 123 im Rasteingriff stehen. Auch das proximale Ende des Ansatzes 117 weist Rastnasen 124 auf, die mit dem Durchbruch 123 in Rasteingriff sind.

Zur Aufhebung des Rasteingriffs können die Rastnasen 120, 121 und 124 nach innen eingedrückt werden, wobei die Rastnasen so dimensioniert sind, dass sie in eingedrücktem Zustand ein Verschieben des Ansatzes bzw. des Zapfens in der Hülse bzw. der inneren Ausnehmung erlauben.

Alle Rastnasen sind mit Schrägen versehen, so dass sie bei proximal auf den Ansatz wirkendem Druck automatisch nach innen gedrückt werden, woraufhin dann Ansatz und Zapfen so weit verschoben werden können, dass es zur Abtrennung der Probensammelvorrichtung wie in Fig. 1a' gezeigt kommt.

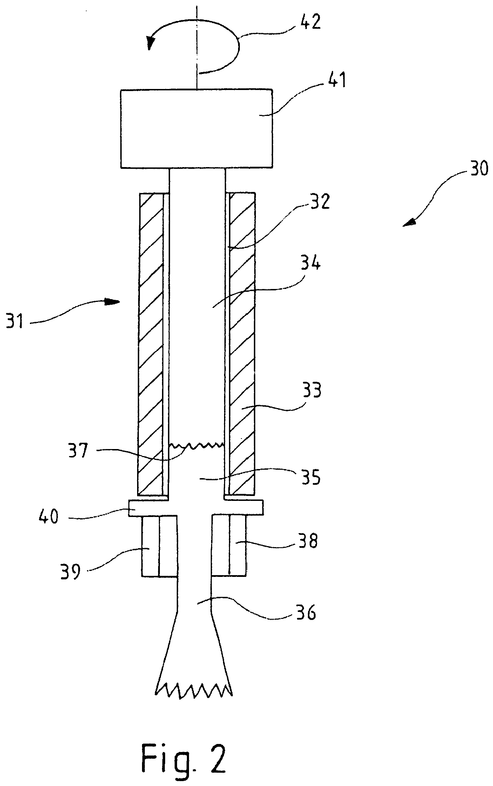

Die Figuren 2-6 zeigen weitere Varianten der Probenahmeeinrichtung.

Die in Fig. 2 gezeigte Probennahmeeinrichtung 30 weist eine Halteeinrichtung 31 mit einer inneren stabförmigen Einrichtung 32 und einer diese umgebenden äußeren Einrichtung in Form eines Röhrchens 33 auf. Die innere stabförmige Einrichtung weist einen distalen Abschnitt 34 und einen proximalen Abschnitt 35 auf, an dessen distalem Ende ein Probensammelbereich 36 angeordnet ist. Der proximale Abschnitt 34 und der distale Abschnitt 35 sind über eine Sollbruchstelle 37 miteinander verbunden. An seinem distalen Ende weist das Röhrchen 33 zwei in Längsrichtung verlaufende Schlitze 38, 39, auf, in die Vorsprünge 40 des distalen Abschnittes 35 eingesetzt sind. An seinem proximalen Ende weist der proximale Abschnitt 34 einen Stellknopf 41 auf, mit dem die stabförmige innere Einrichtung 32 in Richtung des Pfeiles 42 verdreht werden kann. Wird bei einer solchen Verdrehung das äußere Röhrchen 33 fixiert, so wird der proximale Abschnitt 34 der stabförmigen Einrichtung 32 an der Sollbruchstelle 37 von dem in den Schlitzen 38, 39, drehfest fixierten distalen Abschnitt abgedreht und dieser kann mit dem daran angeordneten Probensammelbereich 36 nach unten aus der Halteeinrichtung 31 herausfallen.

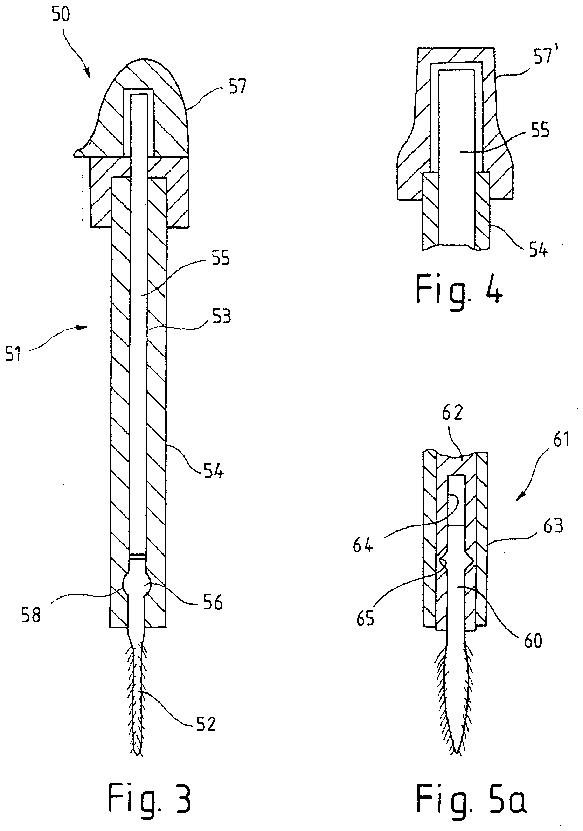

In Fig. 3 ist eine Probennahmeeinrichtung 50 dargestellt, die wiederum eine Halteeinrichtung 51 für einen distalen Probensammelbereich 52 mit einer inneren stabförmigen Einrichtung 53 und einer diese umgebenden röhrchenförmigen äußeren Einrichtung 54 aufweist. Die stabförmige Einrichtung 53 weist wiederum einen proximalen Abschnitt 55 auf, der nach oben aus dem Röhrchen 54 übersteht und einen distalen Abschnitt 56, der den Probensammelbereich 52 trägt. Zwischen distalem Abschnitt 56 und dem proximalen Abschnitt 55 besteht keine Verbindung, d. h. beide Teile liegen bündig fluchtend in der hülsenförmigen äußeren Einrichtung 54.

Gegen ein ungewolltes Verschieben während der Probennahme ist die stäbchenförmige Einrichtung 53 durch eine Kappe 57 gesichert. Der distale Abschnitt 56 ist weiterhin über einen Formschlusseingriff 58 in dem Röhrchen 54 gegen ein Herausfallen etc. gesichert.

Soll nun der Probensammelbereich 52 abgetrennt werden, so wird die Kappe 57 entfernt und die stäbchenförmige Einrichtung 55 so weit in die Hülse 54 eingedrückt, bis der distale Abschnitt 56 vollständig nach unten vorgeschoben ist und aus der Hülse 54 herausfallen kann. Denkbar ist natürlich auch, dass die Hülse 54 gegenüber dem Stäbchen nach oben bewegt wird, wodurch sich dann der gleiche Effekt einstellt.

Fig. 4 zeigt eine Ausgestaltung, bei der eine weiche Kappe 57' vorgesehen ist, die das proximal überstehende Ende der stäbchenförmigen Einrichtung 55 umgibt. Die Kappe 57' ist aus einem weichen Material und erlaubt eine Betätigung ähnlich wie bei einem Kugelschreiber.

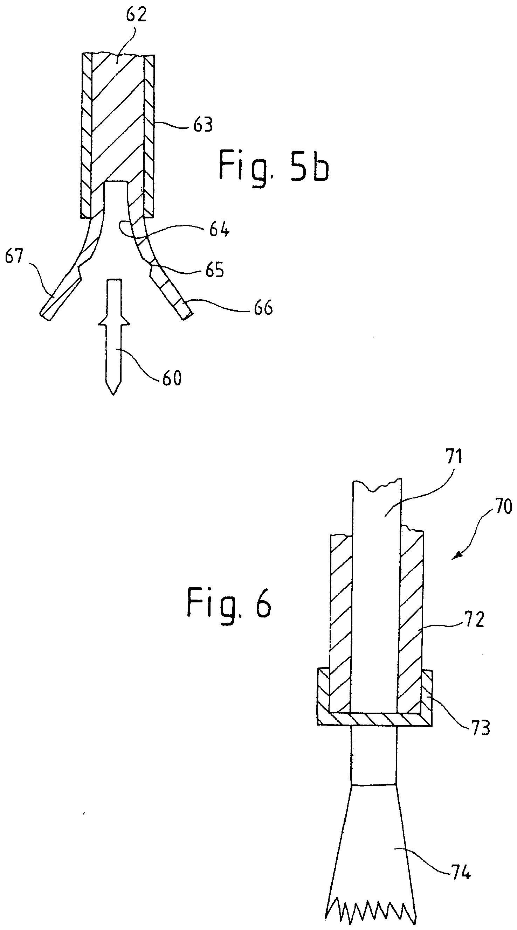

Fig. 5a und 5b zeigen Alternativen für die Aufnahme eines distalen Abschnittes 60 in einer Halteeinrichtung 61. In diesem Fall ist eine innere stäbchenförmige Einrichtung 62 vorgesehen, die von einem Röhrchen 63 umgeben ist. An ihrem distalen Ende ist in der stäbchenförmigen Einrichtung 62 ein in Längsrichtung verlaufender Schlitz 64 vorgesehen, in dem mittels Formschlusseingriff 65 der distale Abschnitt 60 angeordnet ist. Wird nun, wie in Fig. 5b gezeigt, der innere stäbchenförmige Abschnitt 62 nach unten aus dem Röhrchen 63 herausgeschoben, so klappen die den Schlitz 64 begrenzenden Abschnitte 66 und 67 der inneren stabförmigen Einrichtung nach außen und geben die Formschlussverbindung mit dem distalen Abschnitt 60 frei.

Eine weitere Variante schließlich sieht eine Halteeinrichtung 70 die wiederum eine innere Einrichtung 71 in Form insbesondere inneres Stäbchen aufweist, und eine äußere, parallel dazu ausgerichtete und das Stäbchen zumindest teilweise umgebende Einrichtung 72. Äußere Einrichtung 72 und Stäbchen 71 sind an ihrem distalen Ende im Presssitz in eine Aufnahme 73 eingesetzt, die an ihrem anderen Ende einen Probensammelbereich 74 trägt. Wird nun durch Relativbewegung zwischen innerer und äußerer Einrichtung die äußere Einrichtung 72 aus der Aufnahme 73 herausgezogen, so ist die Verbindung zwischen Halteeinrichtung und Aufnahme aufgelöst und die Aufnahme wird mit dem daran befestigten Probensammelbereich automatisch abgetrennt.

## Patentansprüche

1. Probennahmeeinrichtung (10) für insbesondere DNA-haltige Proben mit einem Probensammelbereich (11), der an dem freien Ende einer länglichen Halteeinrichtung (12) vorgesehen ist, wobei der Probensammelbereich (11) von der Probennahmeeinrichtung abtrennbar ausgebildet ist, **dadurch gekennzeichnet, dass** die Halteeinrichtung (12) in Längsrichtung gesehen einen proximalen Abschnitt (13) mit einer sich von seinem distalen Ende mindestens über einen Längenbereich in proximale Richtung erstreckenden inneren Ausnehmung (15) und eine distal fluchtend zu der Ausnehmung (15) ausgerichtete Hülse (14) aufweist, wobei der proximale Abschnitt (13) und die distale Hülse (14) über einen mit geringem Spiel in die innere Ausnehmung (15) und die Hülse (14) eingesetzten Zapfen (16) miteinander verbunden sind, wobei die Maße des Zapfens (16), der Hülse (14) und der inneren Ausnehmung (15) so aufeinander abgestimmt sind, dass im Normalbetrieb eine stabile Verbindung des proximalen Abschnitts mit der Hülse hergestellt ist und dass der Probensammelbereich (11) an einem länglichen Ansatz (17) angeordnet ist, dessen freies Ende wiederum mit geringem Spiel in das distale Ende der Hülse (14) eingesetzt ist, wobei die Längen der inneren Ausnehmung (15), der Hülse (14), des Zapfens (16) und des Ansatzes (17) so aufeinander abgestimmt sind, dass der Zapfen (16) bei proximal gerichteter Verschiebung des Ansatzes (17) relativ zur Hülse (14) vollständig in die Ausnehmung einschiebbar ist.

2. Probenahmeeinrichtung nach Anspruch 1, **dadurch gekennzeichnet, dass** die Abmessungen der inneren Ausnehmung (15), der Hülse (14), des Zapfens (16) und des Ansatzes (7) so aufeinander abgestimmt sind, dass Zapfen (16) und/oder Ansatz (7) in der Hülse (14) und/oder inneren Ausnehmung (15) im Presssitz aufgenommen sind.

3. Probenahmeeinrichtung nach Anspruch 1, **dadurch gekennzeichnet, dass** Zapfen und/oder Ansatz in der Hülse und/oder inneren Ausnehmung mittels einer Formschlussverbingung, insbesondere einer Rastverbindung gesichert sind.

4. Probenahmeeinrichtung nach Anspruch 1, **dadurch gekennzeichnet, dass** ein die relative Verschiebung des Zapfens in der inneren Ausnehmung begrenzendes Sperrelement (19) vorgesehen ist.

5. Probenahmeeinrichtung nach dem Oberbegriff des Anspruchs 1, **dadurch gekennzeichnet, dass** die Halteeinrichtung (31, 51, 61) eine innere stabförmige Einrichtung (32, 53, 62, 71) aufweist, an deren distalem Ende der Probensammelbereich (36, 52, 74) angeordnet ist, sowie eine äussere Einrichtung (33, 54, 72), die die innere Einrichtung zumindest teilweise umgibt und relativ dazu bewegbar ist, wobei der Probensammelbereich (74) entweder lösbar mit der Halteeinrichtung (70) verbunden und durch Relativbewegung von innerer und äusserer Einrichtung davon abtrennbar ist, oder die innere Einrichtung (32, 53, 62) Abschnitte (34, 35; 55, 56; 62, 60) aufweist, von denen der eine den Probensammelbereich trägt und durch Relativbewegung von innerer und äußerer Einrichtung von der Halteeinrichtung abtrennbar ist.

6. Probenahmeeinrichtung nach Anspruch 5, **dadurch gekennzeichnet, dass** die innere Einrichtung einen proximalen Abschnitt (34) und einen damit über eine Sollbruchstelle (37) verbundenen distalen Abschnitt (35) aufweist, der den Probensammelbereich (36) trägt und eine äussere Einrichtung (33) in Form eines die innnere Einrichtung teilweise umgebenden Röhrchens vorgesehen ist, in dem der distale Abschnitt in Längsrichtung verschiebbar und gegen Verdrehung gesichert und der proximale Abschnitt verdrehbar aufgenommen ist.

7. Probenahmeeinrichtung nach Anspruch 5, **dadurch gekennzeichnet, dass** die innere Einrichtung (53) einen distalen, den Probensammelbereich (52) tragenden Abschnitt (56) und einen daran angrenzend angeordneten aber nicht damit verbundenen proximalen Abschnitt (55) aufweist, wobei eine die Trennstelle zwischen distalem und proximalem Abschnitt umgebende äussere Einrichtung (54) vorgesehen ist und der proximale Abschnitt nach oben aus der äusseren Einrichtung herausragt.

8. Probenahmeeinrichtung nach Anspruch 5, **dadurch gekennzeichnet, dass** die distalen Enden der inneren (71) und der äusseren (72) Einrichtung der Halteeinrichtung (70) im Presssitz in eine Aufnahme (73) eingesetzt sind, die an ihrem anderen Ende den Probensammelbereich (74) trägt, wobei innere und äussere Einrichtung in Längsrichtung relativ zueinander verschiebbar ausgebildet sind und durch entsprechende Relativbewegung die Verbindung zwischen Halteeinrichtung (70) und Aufnahme (73) trennbar ist.
